Europäisches Patentamt

European Patent Office

Office européen des brevets

(19)

(11) Veröffentlichungsnummer : **0 153 376**
**B1**

(12) **EUROPÄISCHE PATENTSCHRIFT**

(45) Veröffentlichungstag der Patentschrift :
**11.01.89**

(21) Anmeldenummer : **84903167.9**

(22) Anmeldetag : **16.08.84**

(86) Internationale Anmeldenummer :
**PCT/DE 84/00171**

(87) Internationale Veröffentlichungsnummer :
**WO/8500744 (28.02.85 Gazette 85/05)**

(51) Int. Cl.⁴ : **A 61 F   2/12**

(54) **KUNSTSTOFF-IMPLANTAT INSBESONDERE FÜR DIE IMPLANTATION IN DIE BRUSTWAND DES MENSCHEN.**

(30) Priorität : **17.08.83 DE 3329733**

(43) Veröffentlichungstag der Anmeldung :
**04.09.85 Patentblatt 85/36**

(45) Bekanntmachung des Hinweises auf die Patenterteilung : **11.01.89 Patentblatt 89/02**

(84) Benannte Vertragsstaaten :
**DE FR GB NL**

(56) Entgegenhaltungen :
**SU--A--   997 639**
**US--A--  3 366 975**
**US--A--  3 453 194**
**Rote Liste, Seite 19010**

(73) Patentinhaber : **Reinmüller, Johannes, Dr. med.**
**Eberhardtstrasse 40**
**D-7900 Ulm (DE)**

(72) Erfinder : **Reinmüller, Johannes, Dr. med.**
**Eberhardtstrasse 40**
**D-7900 Ulm (DE)**

(74) Vertreter : **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel**
**Postfach 860820**
**D-8000 München 86 (DE)**

EP 0 153 376 B1

## Beschreibung

Ziel der Implantation von Kunststoffen in die Brustwand des Menschen durch einen chirurgischen Eingriff ist stets der Aufbau einer unterentwickelten weiblichen Brust oder der Wiederaufbau einer durch vorausgegangene ärztliche Maßnahmen verlorengegangenen weiblichen Brust. Hierbei wird ein von der äußeren Form und von der Konsistenz den natürlichen Gegebenheiten entsprechendes Ergebnis angestrebt.

Zur Zeit sind von verschiedenen Herstellern Kunststoff-Implantate erhältlich, die die Forderungen nach äußerer Form und Konsistenz weitgehend erfüllen. Die verwendeten Kunststoffe — es handelt sich überwiegend um Silicone — sind in der Regel nicht gesundheitsschädlich und heilen im allgemeinen in den menschlichen Organismus ein.

Wie jedoch aus der Fachliteratur, z. B. aus Biomaterials in Reconstructive Surgery, L. R. Rubin The C. V. Mosby Company, St. Louis-Toronto-London 1983, insbesondere Kapitel 33, 36 und 38, hervorgeht, erzeugen diese Kunststoff-Implantate nach ihrer Einheilung in den Organismus in einem hohen Prozentsatz (bis 75 %!) Reaktionen in dem sie umgebenden Gewebe (Muskulatur oder Fettgewebe), wobei die Zellen des Bindegewebes eine bindegewebige Kapsel um das Implantat herum bilden. Diese bindegewebige Kapsel neigt zur Schrumpfung. Durch die Kapselschrumpfung wird ein allseitiger Druck auf das Implantat ausgeübt, es verformt sich zur Kugel und die ursprünglich vorhandene Verformbarkeit bzw. Elastizität geht verloren, d. h. wird von fremden Kräften — denen der Bindegewebskapsel — überlagert. Bei der äußerlichen Betrachtung erscheint die Brust unnatürlich geformt und bei der Betastung zeigt sich eine unnatürliche, prall-elastische Konsistenz.

Die Ursachen für die oben beschriebene Kapselbildung mit nachfolgender Kapselschrumpfung sind wissenschaftlich noch nicht erforscht. Ein kausales Eingreifen in die Entwicklung ist deshalb nicht bekannt.

Die Bemühungen zur Verhinderung bzw. zur Behandlung der Kapselbildung bzw. Kapselschrumpfung beschränken sich zur Zeit auf folgende Maßnahmen :

a) Kapselsprengung manuell von außen, Massage

b) chirurgische Kapselsprengung (operativer Eingriff)

c) Gabe von systemisch wirksamen Pharmaka (Tabletten etc.) wie Vitamin E.

d) Anbringen von speziellen Schutzschichten in den Implantathüllen, die häufig aus Polytetrafluorethylen bestehen, zur Verhinderung von Siliconaustritt (da verschiedene Hypothesen hierin die Ursache für die unerwünschte Entwicklung sehen)

e) Gabe von Cortisol oder seinen Abkömmlingen (Nebennierenrindenhormone) in das Implantatlager, d. h. zwischen die Oberfläche des Implantates und die innere Schicht der Bindegewebskapsel. Das Hormon kann entweder in Form einer wäßrigen Lösung in das Innere des Implantates gegeben werden und diffundiert aufgrund des relativ niedrigen Molekulargewichtes an die Oberfläche des Implantates, oder das Implantat ist von einer doppelschichtigen Hülle umgeben. Das Hormon wird in diesem Falle zwischen die beiden Schichten gegeben und gelangt durch Diffusion durch die äußere Hülle an die Oberfläche des Implantates. In beiden Fällen muß der Zusatz des Hormons vor dem Einsetzen des Implantates in den Organismus vorgenommen werden.

Eine Wirksamkeit der bekannten Verfahren a) bis d) ist bisher nicht erwiesen. Das Verfahren e) verhindert bei ausreichend hoher Dosierung der Wirksubstanz die Kapselbildung bzw. -schrumpfung, ist dann jedoch mit erheblichen Nebenwirkungen des Hormons am Ort und systemisch im Organismus verbunden, d. h. es treten in einem hohen Prozentsatz zusätzliche Gesundheitsstörungen auf.

Der Erfindung liegt die Aufgabe zugrunde, die oben angegebenen Nachteile, insbesondere eine Kapselbildung bzw. -schrumpfung um Kunststoff-Implantate, besonders im Brustwandgewebe zu verhindern, ohne daß die Nachteile (Gesundheitsschäden) des Verfahrens e) auftreten.

Diese Aufgabe wird erfindungsgemäß gelöst durch die Verwendung von Heparin zur Herstellung einer die Ausbildung einer zum Schrumpfen neigenden Bindegewebskapsel verhindernden Heparin-Beschichtung auf einem Kunststoff-Implantat für die Einpflanzung in bluttrockene Körperhöhlen.

Heparin ist eine natürlicherweise im menschlichen Organismus vorkommende Substanz mit polymerem Charakter. Sie wird zu anderen Zwecken, nämlich zur Hemmung der Blutgerinnung, in der Medizin eingesetzt und von verschiedenen Herstellern kommerziell aus tierischem Gewebe gewonnen.

Es ist sehr wichtig, daß Brustimplantate nur in bluttrockenen Höhlen implantiert werden. Mangelhafte Blutstillung bei der Schaffung der Implantathöhle durch den Chirurgen führt in der Regel zur Nachblutung in das Implantatlager und zur Hämatombildung, einer sehr unerwünschten Komplikation.

Überraschenderweise erweist sich Heparin bei der erfindungsgemäßen Anwendung als potenter Hemmstoff der Bindegewebsneubildung und verhindert somit eine überschießende Bindegewebsneubildung bzw. es verhindert die Kapselbildung im unerwünschten Maße.

Diese Wirkung des Heparins war nicht vorhersehbar, da Heparin bisher klinisch ausschließlich zur Hemmung der Blutgerinnung eingesetzt oder vorgeschlagen wurde (siehe z. B. US-A-3 453 194). Wie sich jetzt durch die Erfindung herausstellt, gelingt es jedoch, Heparin zur Verhinderung unerwünschter Bindegewebsreaktionen einzusetzen.

Die Erfindung ist nicht auf Brustimplantate beschränkt, auch wenn diese bevorzugt werden. Ganz analoge Probleme treten auch bei Kunststoffimplantaten in anderen Positionen auf wie Wadenimplantaten, künstliche Beugesehnen an der Hand aus Silikon, spezielles chirurgisches Nahtmaterial aus Kunststoffäden, Trachealkanülen und dergleichen. In allen diesen Fällen gelingt es mit dem erfindungsgemäßen Implantat, die Ausbildung von Fremdkörpergranulomen zu verhindern.

Die Nebenwirkungen von Heparin sind dosisabhängig und betreffen nahezu ausschließlich das Blutgerinnungssystem.

Der Abbau von Heparin erfolgt in der Leber. Ein weiterer Vorteil des Heparin ist darin zu sehen, daß es nicht plazentagängig ist, d. h. im Falle einer Schwangerschaft keine Schädigung der Leibesfrucht hervorrufen kann. Einflüsse des Heparins auf den Hormonhaushalt des menschlichen Organismus sind nicht bekannt. Heparin weist somit erhebliche Vorteile gegenüber Nebennierenrindenhormonen bezüglich des erfindungsgemäßen Einsatzes auf.

Für die Basisimplantate kommen die hierfür bekannten und üblicherweise angewendeten Kunststoffe bzw. Polymere in Betracht, insbesondere die überwiegend gebräuchlichen Silikone. Auf der Oberfläche dieser Kunststoffe wird zur erfindungsgemäßen Herstellung des Implantats das Heparin nach einer der hierfür bekannten chemischen oder physikalischen Methoden so fixiert, daß der überwiegende Teil der Oberfläche, vorzugsweise die gesamte Oberfläche, von Heparin bedeckt ist.

Eine chemische Fixierung gelingt nach den bekannten Methoden der Immobilisierung von Heparin und anderen chemisch ähnlichen Substanzen an festen Trägermaterialien. Unter den geeigneten Methoden seien die Haftungsvermittlung mittels quaternärer Ammoniumsalze, vorzugsweise mit Tridodecylmethylammoniumchlorid (TDMAC), kovalente Fixierung nach den für die Immobilisierung von Naturstoffen, welche Hydroxylgruppen oder Sulfonatgruppen aufweisen, bekannten Methoden, Vernetzung des Heparins auf der Oberfläche durch bifunktionelle Reagenzien wie Glutardialdehyd, Diepoxyde und dergleichen sowie ionische Bindung durch Einführung von kationischen Gruppen an der Kunststoffoberfläche, die mit den Sulfonatgruppen des Heparins unter Salzbildung reagieren können, als Beispiele aufgeführt.

Physikalisch kann die Fixierung erfolgen durch Anordnung einer porösen Hülle um das eigentliche Implantat, wobei das Heparin, welches in gelöster oder in fester Form vorliegen kann, im Raum zwischen Kunststoffoberfläche und poröser Hülle angeordnet ist. Die äußere Kunststoffhülle muß dabei hinsichtlich ihrer Porosität so beschaffen sein, daß das Heparin, welches ein Molekulargewicht von einigen Tausend bis etwa 17.000 Dalton aufweist, passieren kann. Da die Molekulargewichtsausschlußgrenzen der handelsüblichen Dialysemembranen bekannt sind, lassen sich geeignete Hüllen ohne weiteres auswählen. Bei dieser Ausführungsform kann das Heparin entweder direkt zwischen der porösen Hülle und der Kunststoffoberfläche bzw. im Kunststoff selbst angeordnet sein oder sich zwischen der äußeren porösen Hülle und einer weiteren Innenhülle, die den eigentlichen Kunststoff einschließt, befinden. Liegt das Heparin hierbei in Lösung unter der porösen Hülle vor, so verwendet man als Lösungsmittel zweckmäßig eine Substanz höheren Molekulargewichts, welche durch die Poren in etwa gleicher Weise zurückgehalten wird wie das Heparin. Beispiele sind höhere Polyethylenglykole, Polyvinylpyrrolidone und dergleichen.

Da heparinisierte Oberflächen aus Vorrichtungen, die von Blut durchflossen werden, zur Verhinderung der Thrombenbildung bekannt sind, stehen bewährte Methoden zur Verfügung.

Die beigefügte Zeichnung zeigt in

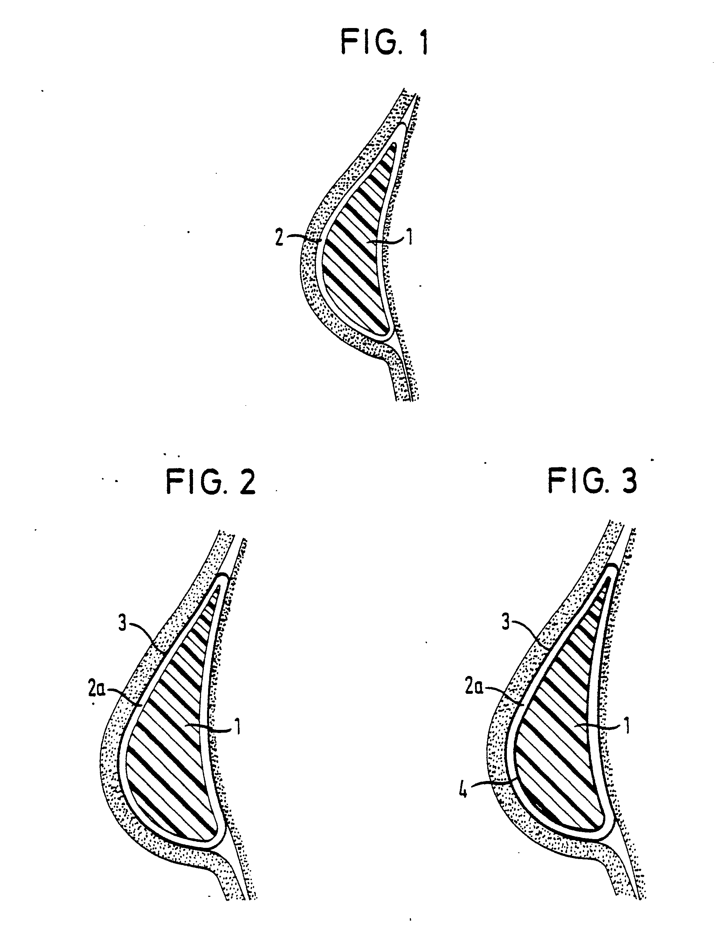

Fig. 1 schematisch einen Schnitt durch ein bereits implantiertes Brust-Implantat aus Kunststoff (1), welches von einer Heparinschicht (2) bedeckt ist. In

Fig. 2 wird eine andere Ausführungsform der Erfindung im Schnitt dargestellt, bei der der Kunststoffkörper (1) von einer Heparinlösung (2a) umgeben ist, die nach außen hin durch eine poröse Membran (3) abgeschlossen wird. Eine weitere Ausbildung der Erfindung zeigt

Fig. 3, die der Fig. 2 entspricht, jedoch zusätzlich noch zwischen Kunststoffkörper (1) und Heparinlösung (2a) eine Kunststoffhülle (4) aufweist.

Die Einbringung des erfindungsgemäß hergestellten Implantats erfolgt operativ gemäß folgender Technik:

Desinfektion der Haut und steriles Abdecken, Hautincision in der Achselhöhle oder in der sog. Inframammarfalte, Durchtrennen des Unterhautfettgewebes bis zur bindegewebigen Hülle des Brustmuskels (M. pectoralis major). Es wird nun eine Höhle geschaffen entweder oberhalb des Muskels — also zwischen Muskel und Fettgewebe — oder unterhalb des Muskels, d. h. zwischen den Rippen und der darüberliegenden Muskulatur. Die neu geschaffene Höhle wird inspiziert und Blutungen werden gestillt, zumeist werden verletzte Blutgefäße durch spezielle Geräte elektrisch erhitzt (Diathermie), so daß das Blut in ihnen gerinnt. Sobald Bluttrockenheit in der Höhle erreicht ist, wird das erfindungsgemäße Implantat unter Beachtung der Sterilität eingebracht. Die Wunde wird mit chirurgischen Nähten in Schichten verschlossen. Eingehend ist diese Technik beschrieben in Chir. Plastica 6, 87-93 (1981). .

Das Heparin kann im Rahmen der Erfindung teilweise oder ganz durch andere Heparinoide bzw. dem Heparin chemisch verwandte Glycosaminoglykane ersetzt werden.

Durch die Erfindung wird es möglich, die Reaktionen des Bindegewebes, die nach dem Implantieren von Kunststoffen in bluttrockene Körperhöhlen, insbesondere in die Brustwand des Menschen, auftreten können und zu Mißerfolgen bei der Behandlung von Patienten führen können, zu hemmen. Hierdurch würde der Erfolg bei entsprechenden Behandlungen statistisch prozentual verbessert. Die Gefahren zusätzlicher Gesundheitsschädigungen, wie bei anderen Verfahren, wird erheblich gemindert.

Die Wirksamkeit der Erfindung wurde durch tierexperimentelle Studien mit Laborratten bestätigt. Die Tiere wurden narkotisiert und erhielten zufällig verteilt rechts und links, sowie links und rechts durch kleine Schnitte an der Brustwand oder am Rücken kleine ihrer Größe entsprechende Silikon-Implantate in das Subkutangewebe, wobei eine Hälfte der Implantate im Inneren Heparin enthält, die andere Hälfte ohne Heparin bleibt. Die Verteilung der Implantate mit/ohne Heparin rechts/links erfolgte rein zufällig. Jedes Tier erhielt somit mindestens zwei Implantate. Die Tiere wurden 4, 6, 8 Wochen usw. unter gleichen Bedingungen gehalten. Danach wurden in Narkose die Implantate palpatorisch beurteilt. Dann wurden die Implantate und das sie umgebende Bindegewebe entfernt und mikroskopisch beurteilt. Es wurde dabei festgestellt, daß immer dann, wenn das Implantat Heparin enthielt, im Vergleich zur Kontrollgruppe nur eine geringe Kapselbildung um die Implantate stattfand.

**Patentansprüche**

1. Verwendung von Heparin zur Herstellung einer die Ausbildung einer zum Schrumpfen neigenden Bindegewebskapsel verhindernden Heparin-Beschichtung auf einem Kunststoff-Implantat für die Einpflanzung in bluttrockene Körperhöhlen.

2. Verwendung von Heparin nach Anspruch 1, dadurch gekennzeichnet, daß die Fixierung des Heparins, Heparinoids und/oder dem Heparin chemisch verwandten Glycosaminoglycan durch Anordnung einer porösen Hülle um das eigentliche Implantat erfolgt, so daß das Heparin, Heparinoid und/oder dem Heparin chemisch verwandte Glycosaminoglycan im Raum zwischen Kunststoffoberfläche und poröser Hülle angeordnet ist.

3. Verwendung von Heparin nach Anspruch 1, dadurch gekennzeichnet, daß das Heparin, Heparinoid und/oder dem Heparin chemisch verwandte Glycosaminoglycan kovalent an die Oberfläche des Kunststoff-Implantates gebunden wird.

4. Verwendung von Heparin nach Anspruch 1, dadurch gekennzeichnet, daß das Heparin, Heparinoid und/oder dem Heparin chemisch verwandte Glycosaminoglycan ionisch an die Oberfläche des Implantates gebunden wird.

5. Verwendung von Heparin nach Anspruch 1, dadurch gekennzeichnet, daß das Heparin, Heparinoid und/oder dem Heparin chemisch verwandte Glycosaminoglycan durch Vernetzung an der Implantat-Oberfläche fixiert wird.

**Claims**

1. Use of heparin for the production of a heparin coating, preventing, the formation of a connective tissue capsule tending to shrinkage, on a synthetic material implant for the implantation into blood-dry body cavities.

2. Use of heparin according to claim 1, characterised in that the fixing of the heparin, heparinoid and/or glycosaminoglycan chemically related to heparin takes place by arrangement of a porous covering around the actual implant so that the heparin, heparinoid and/or glycosaminoglycan chemically related to heparin is arranged in the space between synthetic material surface and porous covering.

3. Use of heparin according to claim 1, characterised in that the heparin, heparinoid and/or glycosaminoglycan chemically related to heparin is covalently bound to the surface of the synthetic material implant.

4. Use of heparin according to claim 1, characterised in that the heparin, heparinoid and/or glycosaminoglycan chemically related to heparin is bound ionically to the surface of the implant.

5. Use of heparin according to claim 1, characterised in that the heparin, heparinoid and/or glycosaminoglycan chemically related to heparin is fixed by cross-linking on the implant surface.

**Revendications**

1. Utilisation d'héparine en vue de former, sur un implant en matière synthétique destiné à être implanté dans des cavités corporelles sans épanchement sanguin, un revêtement d'héparine qui empêche le développement d'une capsule de tissu conjonctif ayant tendance à la contraction.

2. Utilisation d'héparine selon la revendication 1, caractérisée par le fait que la fixation de l'héparine, de l'héparinoïde et/ou d'un glycosaminoglycane chimiquement apparenté à l'héparine a lieu par mise en place d'une enveloppe poreuse autour de l'implant proprement dit, de sorte que l'héparine, l'héparinoïde et/ou le glycosaminoglycane chimiquement apparenté à l'héparine se trouve dans l'espace situé entre la surface de la matière synthétique et l'enveloppe poreuse.

3. Utilisation d'héparine selon la revendication 1, caractérisée par le fait que l'héparine, l'héparinoïde et/ou le glycosaminoglycane chimiquement apparenté à l'héparine est combiné par covalence avec la surface de l'implant en matière synthétique.

4. Utilisation d'héparine selon la revendication 1, caractérisée par le fait que l'héparine, l'héparinoïde et/ou le glycosaminoglycane chimiquement apparenté à l'héparine est combiné ioniquement avec la surface de l'implant.

5. Utilisation d'héparine selon la revendication 1, caractérisée par le fait que l'héparine, l'héparinoïde et/ou le glycosaminoglycane chimiquement apparenté à l'héparine est fixé par réticulation à la surface de l'implant.

# FIG. 1

# FIG. 2

# FIG. 3